# EUROPEAN PATENT APPLICATION

(11) **EP 1 955 710 A1**
(43) Date of publication of application: **13.08.2008**
(21) Application number: 07002778.4
(22) Date of filing: 09.02.2007
(51) Int. Cl.: A61K 47/48

(54) **Aqueous composition comprising chitosan and an acidic drug**

(71) Applicant: The Jordanian Pharmaceutical Manufacturing Co., 11710 Naor (JO)
(72) Inventor: Badwan, Adnan, 11185, Amman (JO); Al-Remawi, Mayyas, 13713, Russiefa (JO)
(74) Representative: Winkler, Andreas Fritz Ernst

(57) **Abstract**

The present invention relates to a composition comprising an aqueous solvent and a conjugate of at least one hydrophilic polymer having a primary amine functional group, a derivative or monomer thereof and at least one acidic drug; a method for its preparation and use thereof.

## Description

The present invention relates to an aqueous composition comprising an acidic drug and a hydrophilic polymer having a primary amine functional group.

Oral pharmaceutical dosage forms are administered to the patient in the form of a solid or liquid. Liquid forms are more suitable for infants, children and older persons who are unable to swallow solid dosage forms. Oral liquid pharmaceutical dosage forms may be provided as solutions, emulsions and suspensions.

Many active ingredients, such as some non-steroidal anti-inflammatory drugs, possess an irritant or unpleasant taste. The unpleasant taste characteristic of an active agent is an extremely important factor in the formulation of liquid pharmaceutical dosage forms.

The prior art methods for taste masking provide a taste mask liquid in suspension form. These methods were utilizing a mechanical barrier by coating the active material particles with an impermeable enteric coat. Then, the coated particles are dispersed in the formula medium to form a suspension liquid.

For example, US 5,599,556 disclosed liquid formulations where the active ingredient is coated with a single outer polymeric coating derived from prolamine cereal grain proteins and a plasticizing agent. The coatings are designed to rapidly degrade once the composition leaves the mouth. US 6,586,012 provides a liquid composition for oral administration comprising a pharmaceutically active medicament coated with a taste masking effective amount of polymer blend of (a) dimethylaminoethyl methacrylate and neutral methacrylic acid ester and (b) a cellulose ester, in an aqueous vehicle.

Chitosan (poly (N-acetylglycosamine)) is partially deacetylated chitin which is one of the most abundant polysaccharides in nature second only to cellulose. It has a sugar backbone consisting of β-1,4 linked glucosamine with a high degree of N-acetylation (70-90% N-acetylglucosamine and 10-30% D-glucose units), a structure very similar to that of cellulose; the only difference being the replacement of the hydroxyl by amino groups.

Chitosan is considered as weak base due to the presence of primary amine group. Thus, it undergoes the typical neutralization reactions of alkaline compounds. Chitosan is insoluble in neutral or alkaline aqueous media. Nearly all aqueous acids dissolve chitosan, some of them are relatively safe for use which allow formation of solutions suitable for making gels.

Acetic acid has been mostly used as a standard solvent for chitosan solutions. Chitosan reacts readily with most aldehydes to form imines. Formaldehyde and gluteraldehyde are considered as cross-linking agents for chitosan. Acyl chlorides react vigorously with chitosan to form the corresponding amide derivatives. Chitosan as it is a natural substance and highly available in nature is inexpensive, non-toxic, biodegradable, biocompatible when compared with other polymers.

Chitosan is considered to be non-digestible by human when taken by oral route, this is due to lack of chitosanases, which are present in some bacteria.

Chitosan acquires a positive charge "cationic polymer" when exposed to acid because of protonation of primary amine group. It coagulates, if a negatively charged molecule is added to its solution, e.g. sodium alginate, sulfate and phosphate form conjugates with chitosan. Pharmaceutical uses of chitosan are very numerous. The scientific and medical literature lists hundreds of industrial, medical, and dietary applications for chitosan. These include protection of sensitive drugs from the deactivating enzymes, preparation of artificial cells, using chitosan to trap hemoglobin, promotion of bone repair, burn dressing and contact lens material.

S.T.P. Pharma in Jan-Feb 2000 published a complete thematic issue on "chitosan in drug delivery systems". Also, there are several review articles on chitosan as a drug carrier, such as W. Paul, C. Sharma. Chitosan, a drug carrier for the 21st century: a review. STP Pharma Sci.,10, pp 5-22, 2000; F. Olivia, P. Buri., R. Gury. Chitosan: A unique Polysaccharide for Drug Delivery: a review. Drug Dev. Ind.Pharm. 24, pp 979-993, 1998.

It is an object of the present invention to provide a pharmaceutical composition, preferably a solution, which can be orally administered but avoids the irritant or unpleasant taste of specific drug contained in that composition.

Additionally, a method for its preparation and uses thereof shall be provided.

The first object is achieved by a composition comprising an aqueous solvent and a conjugate of at least one hydrophilic polymer having a primary amine functional group, a derivative or monomer thereof and at least one acidic drug.

Preferably, the polymer is chitosan or the monomer is glucosamine.

In this context, it is also preferred that the acidic drug contains an acidic functional group selected from the group consisting of carboxylic, sulfonic, nitric, phosphoric group, or a salt thereof.

Most preferably the acidic drug is insoluble in water.

The acidic drug may be selected from beta lactam antibiotics, quinolone antibiotics, sulfonamide, nonsteroidal anti-inflammatory drugs, anti-hyperlipidimic drugs, psychostimulants, prostaglandin vasodilators, antidepressants, anticonvulsive agents, hemostatic agents, antituberculosis agents, hepatic protectant agents, flavoring agents, sweeteners, antispasmodic agents, anti-neoplastic agents, antiseptic agents, anti-inflammatory cortisone drugs, hypoglycemic drugs, anti-arrythmetic agents, antihypertensive agents, anti-allergy agents, emollients, agents for gastrointestinal disorders and anti-infective.

In this regard, the acidic drug may be selected from cephalosporins, penicillins, nalidixic acids, ciprofloxacin, acediasulfone, amfenac, diclofenac, ibuprofen, indomethacin, acetyl salicylic acid, clinadac, naproxen, mefenamic acid, fosfomycin, bendazac, acifran, fluvastatin, atorvastatin, aceglutamide, alprostadil, amineptine, gamma aminobutyric acid, gabapentin, valproic acid, aminocaproic acid, aminosalicylic acid, amino acid arginine, aspartic acid, betaine, aspartam, baclofen, bendamustine, bromebric acid, sodium borocaptate, chlorambucil, methotrexate, bismuth subgallate, dodicin, betamethasone, calcium mesoxalate, capobenic acid, captopril, cromolyn sodium, spaglumic acid, docusate sodium, mesalamin and flumequine.

The composition is preferably characterized in that the aqueous solvent has a pH of 4 to 7, preferably 4 to 6.5.

The aqueous solvent may be 0.01 M HCl solution.

The composition may further comprise at least one additional therapeutic active ingredient other than the acidic drug as disclosed above.

Preferably, the composition additionally comprises pharmaceutical excipients selected from the group consisting of sweeteners, coloring agents, preservatives, thickeners and flavoring agents.

According to the invention is also a method for preparing a conjugate of at least one hydrophilic polymer having a primary amine functional group, a derivative or monomer thereof and at least one acidic drug, comprising the steps of reacting the acidic drug to obtain an acid halide ester anhydride or any other intermediate product and reacting the intermediate product with the hydrophilic polymer to form a conjugate via amide bond formation.

Preferably the method is carried out under acidic environment.

The composition may be preferably used for oral administration of the drug or for taste masking of the acidic drug.

Surprisingly it was found that the pharmaceutical composition according to the present invention has a taste masking effect of bitter drugs, probably due to the conjugate formation between the hydrophilic polymer and the acidic drug. Further, it was noted that the drug stability is significantly enhanced. The inventive composition may be prepared in a cost effective and easy way.

Additional features and advantages of the subject-matter of the present invention can be taken from the following detailed description thereof with reference to the accompanied drawings, wherein
Figure 1 illustrates the formation of a conjugate comprising chitosan and ibuprofen;
Figure 2 shows a structure of a water-soluble chitosan-acidic drug conjugate system;
Figure 3 shows UV spectra of ibuprofen (A) and ibuprofen ethanoate (B).
Figure 4 shows IR spectra of chitosan (A) and chitosan ibuprofen conjugate (B).
Figure 5 shows a plasma concentration time profile of ibuprofen, when ibuprofen chitosan solution (amide conjugate) is given to rabbits via oral route in a dose of 90 mg/kg compared to oral ibuprofen solution in a dose of 10 mg/kg; and
Figure 6 shows a diagrammatic presentation for % average taste evaluation for inventive examples and comparative examples.

Ibuprofen represents a good model drug for conjugation with chitosan polymer. Ibuprofen is a non-steroidal anti-inflammatory product. The molecular weight is 206. It is practically insoluble in water. Ibuprofen is rapidly absorbed from GIT after oral administration. Peak plasma levels of 15 to 20 mcg/ml occur about 1 hr after administration of a single 200-mg oral dose in fasting subjects. The serum half-life after a single 200 mg oral dose in fasting subjects was about 1.9hr.

Ibuprofen is an effective pain killer for muscular-skeletal, rheumatic, menstrual and dental pain. It belongs to the group of medicines called "non-steroidal anti-inflammatory drugs" (NSAIDS's). Ibuprofen is known of its oral and gastric irritation effect like most NSAID. This irritation effect results in an unpleasant sensation in the throat if the drug is taken in oral form. In addition, this irritation effect may contribute to the formation of gastric ulcers on long-term use. It is known that the second major cause for ulcers is the irritation of the stomach arising from regular use of NSAID's.

Chitosan has a wide range of molecular weights. Chitosan of large molecular weight of more than 50.000 Da has low water solubility and forms high viscosity solutions in acidic aqueous medium. Low molecular weight chitosans of less than 50.000 Da (weight average) are usually water-soluble and form low viscosity solution. Chitosan having molecular weight ranging from 20.000 to 1.000 may refer to as chitosan oligosaccharides. Chitosan oligosaccharide are the most preferred for the present invention.

The invention describes the use of chitosan and a conjugate forming polymer for masking the local or direct active material effect on the whole gastrointestinal tract including the taste of the active material.

In this invention, the chitosan ibuprofen conjugate mask the taste of ibuprofen. The taste was checked by 6 volunteers, see example 4 below. The plasma concentration time profile of ibuprofen was established to proof the drug release in-vivo using rabbit models, see example 3 below.

Chitosan, especially oligosaccharides thereof are known for their high solubility and oral permeability. The conjugation of an acidic drug, such as ibuprofen, can be easily achieved by different chemical reactions. This can be achieved via conversion of ibuprofen to a suitable activated intermediate that can react with chitosan primary amine groups. This intermediate can be achieved by forming an acid halide, ester, anhydride or any activation process known in the art. Upon reacting activated intermediate with chitosan a drug chitosan conjugate is formed. The conjugate was found to be water-soluble. The drug was detected in the plasma of rabbits upon giving the conjugate in the form of oral solution. The mechanism of oral drug release from the solution is not well established. The conjugate might release the active material upon hydrolizing the conjugate via the liver or gut enzymes. The drug local irritation effect was masked by the process of conjugation.

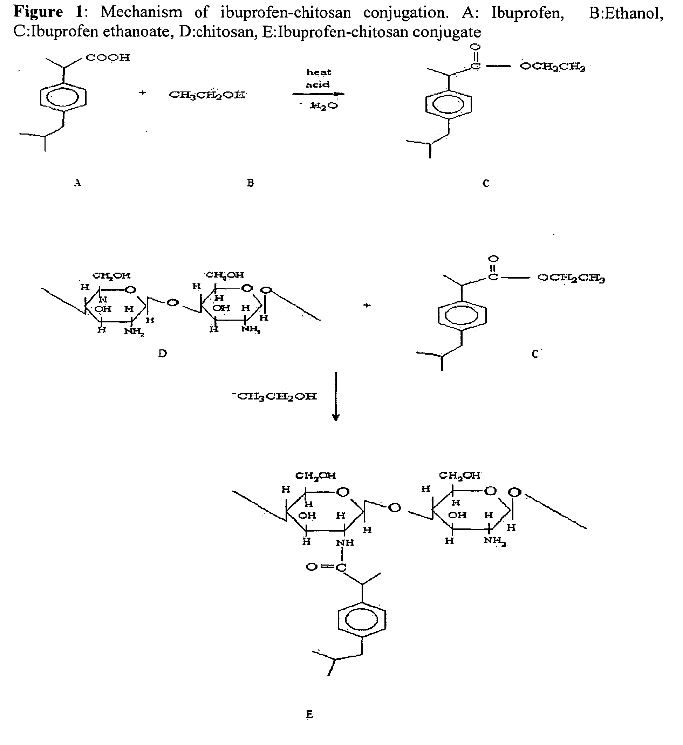

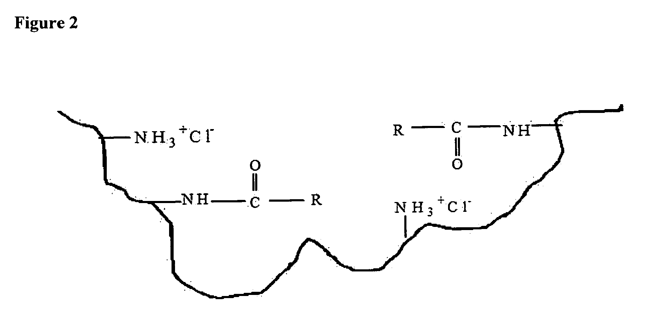

Chitosan-ibuprofen conjugate results through the formation of a covalent amide bond, Fig. 1. The amide bond is formed by chemical interactions during solution formulation. Where, ethanol acts as an intermediate for the formation of the amide link i.e. it helps in the formation of an ibuprofen ester that interact with primary amine groups in chitosan polymer to form the amide bond and leaves the solution as a byproduct which is get rid of by evaporation. Acidic environment aids ibuprofen ester formation. In addition, it helps in acidification of free primary amine groups in chitosan (protonation) and so solubilization in aqueous medium. Ibuprofen ethanoate, water insoluble ester, is mixed with chitosan aqueous solution and forms an emulsion. The end point of conjugation is obtained once a homogenous clear solution is obtained. The incomplete amide bond formation between the drug and chitosan is obvious due to the steric hindrance effect. The free primary amine groups are useful to be used as a source for ionization of the free primary amine groups in the chitosan-drug conjugate by the addition of acidic substance such as hydrochloric acid, Fig. 2. This would increase total polymer solubility. A clear solution is to be formed containing the polymer linked with the active material.

When the solution is taken orally, the amide bond is not cleaved in the mouth due to short duration period and absence of amide cleaving enzymes in the saliva. Thus, the bitter taste of the drug is masked by this way. Free drug is now ready to be absorbed through the small intestine.

Examples on the active material related to the invention are beta lactam antibiotics such as cephalosporins and penicillins. Quinolone antibiotics such as nalidixic acid, ciprofloxacin. Sulfonamide such as acediasulfone. Nonsteroidal anti-inflammatory drugs such as amfenac, diclofenac, ibuprofen, acetyl salicylic acid, clinadac, naproxen, mefenamic acid, fosfomycin, bendazac. Antihyperlipidimic such as acifran, fluvastatin, atorvastatin. Psychostimulant such as aceglutamide. Prostaglandin vasodilator such as alprostadil. Antidepressant such as amineptine, gamma aminobutyric acid, gabapentin. Anticonvulsive agent such as valproic acid. Hemostatic agents such as aminocaproic acid. Anti-tuberculosis such as aminosalicylic acid. Hepatic protectant amino acid arginine, aspartic acid, betaine. Flavoring agents and sweeteners such as aspartam. Antispasmodic agent such as baclofen. Antineoplastic agent such as bendamustine, bromebric acid, sodium borocaptate, chlorambucil, methotrexate. Antiseptic such as bismuth subgallate, dodicin. Anti-inflammatory cortisone drugs such as betamethasone. Hypoglycemic oral drugs such as calcium mesoxalate. Antiarrythmetic agents such as capobenic acid. Antihypertesive agents such as captopril. Antiallergy agents such as cromolyn sodium, spaglumic acid. Emollient such as docusate sodium. GI disorders such as mesalamine. Anti-infective such as flumequine.

### Examples

In all examples the chitosan used was purified and activated by washing of chitosan with n-hexane and then ethanol 96% until a clear spectrum in ethanol is obtained by UV spectrum. Then the purified chitosan was completely dried in oven before use to get rid of organic solvents.

### Example 1

### Conjugation reaction

Chitosan, a natural product obtained mainly from cramps and fish, has a high adsorption affinity towards fatty substances that may attach to its structure during the process of preparation. Thus, purification of chitosan needs the use of organic solvents such as n-hexane and ethanol to get rid of fatty materials or other impurities. These impurities may deactivate the primary amine groups (active sites) present in chitosan. Purification from these impurities may increase the ability for binding to drug molecules.

Ibuprofen (carboxylic acid) was esterified using alcohol (ethanol). Ibuprofen ethanoate (insoluble) was reacted with purified chitosan in an aqueous solution, Fig 1. Slight acidic medium of hydrochloric acid was added to enhance the conjugation process and to solubilize chitosan polymer in aqueous solution. Ibuprofen ethanaote was then reacted with chitosan and a homogenous mixture of Ibuprofen-chitosan conjugate was formed.

### Example 2

### Characterization of conjugation

Infra red spectra were obtained using Fourier transform infra red spectrometer (FTIR). FTIR was performed under room air at room temperature and KBr disk. Samples were placed in an oven at 105°C for 3 hours before doing any measurements to get rid of moisture. Approximately 500 mg of KBr and 5 mg of sample powder were blended with pestle and mortar for 5 min. The sample disk was prepared at a pressure of 9 tons for 21 min.

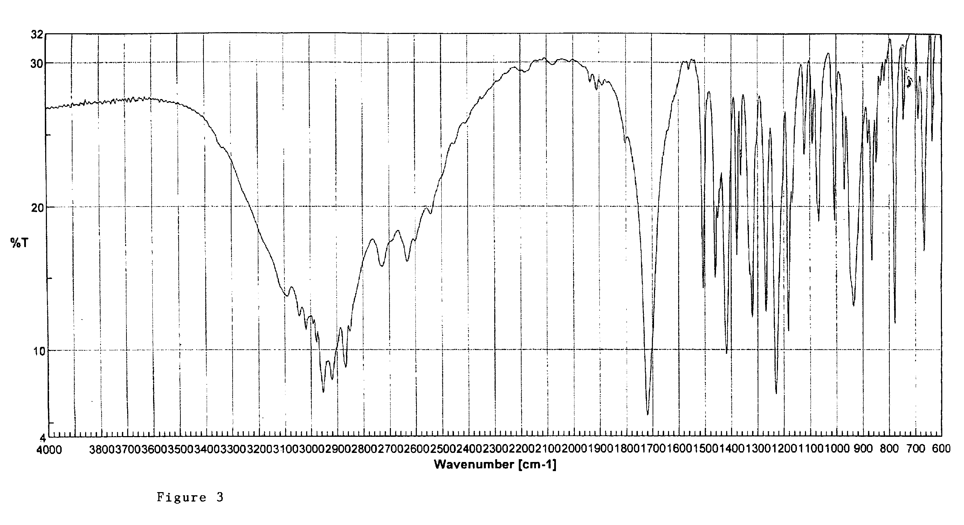

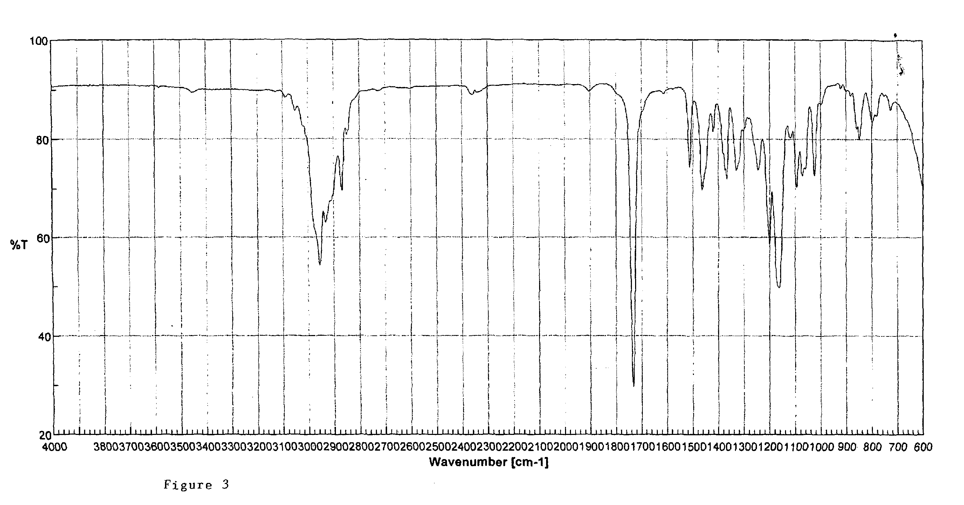

The ultraviolet and infrared spectra of ibuprofen and its ethanoate ester are shown in Fig 3. The spectra differences indicate that there may be a slight difference in the chemical structure. In case of infrared spectra, the large peaks width between 2850-3500 cm-1, which corresponds to hydrogen bonding in ibuprofen carboxylic acid functional groups, decreased tremendously indicating the transformation of the carboxylic acid to ethyl carboxylate of ibuprofen ethanoate ester. A shift in carbonyl functional group for ibuprofen of wave number 1720 to 1740 cm-1 indicates the formation of ibuprofen ethanoate.

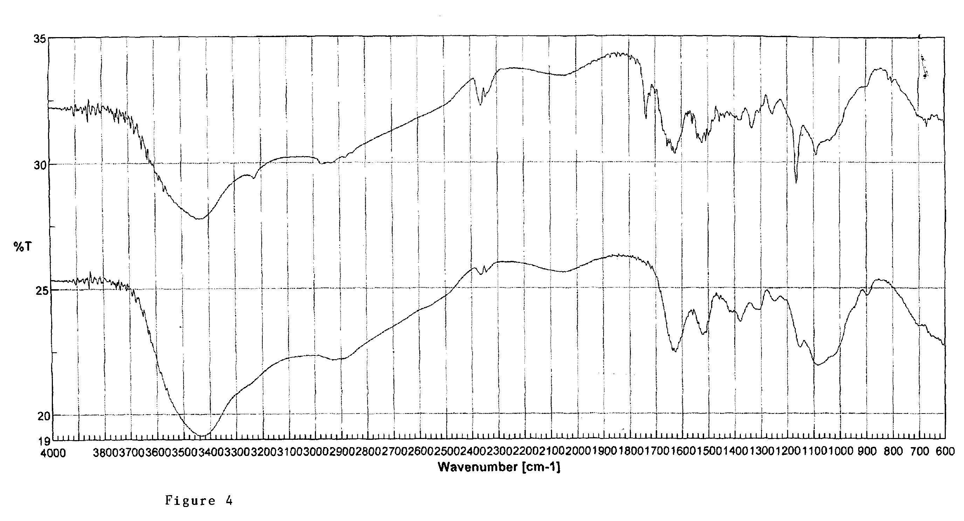

The amide bond formation between ibuprofen and chitosan was indicated by physical conversion of the emulsion into a clear transparent solution. In addition, amide bond formation was indicated by the appearance of the carbonyl band at 1740 cm-1 and the C-O band at 1160 cm-1. Where, these bands were not observed in the IR spectrum of chitosan, Fig 4.

### Example 3

### In vivo evaluation of drug release

Ibuprofen was conjugated to chitosan having weight average molecular weight of less than 3000 as described in example 1, where a clear solution was obtained for controlled release delivery. Ibuprofen solution unconjugated with chitosan was dissolved in water/propylene glycol mixture, where a clear solution was obtained for immediate release delivery.

The conjugate containing ibuprofen and chitosan was given orally to rabbits in a dose of 90 mg of ibuprofen/kg. A reference for immediate release oral solution given with a dose of 10 mg of ibuprofen/kg rabbit was also utilized. Blood samples were withdrawn at specified time interval, centrifuged at 3500 rpm for 10 minutes and placed in a freezer at -20 °C . The samples were analyzed using HPLC method. A simple rapid method of determining the ibuprofen concentration by HPLC was developed. Naproxen was used as an internal standard. *Mobile Phase:* Water: Acetonitrile (40:60), then adjust pH to 2.4 with H3PO4. *Column:* Waters, Symmetry, 5u, C18, and 150*4.6 mm. *Internal STD Stock Solution:* Dissolve 5 mg of Naproxen into 50 ml MeOH. *Injection Loop:* 50ul. *Flow rate:* 1 ml/min. *Wavelength:* 220 nm.

Sample Preparation: Transfer 100 uL of plasma sample to test tube, Add 10 uL of Internal STD Stock solution, Add 0.25 ml of 1 M HCl, Shake for 30 seconds, Add 5 mL of (85:15)(Hexane: Isopropanol) Shake with vortex for 1 min., Centrifuge at 3000 rpm for 10 min., Transfer 4 ml of organic layer to new test tube, Evaporate the organic solvent using an air shower, and Reconstitute with 1 ml mobile phase. The method was evaluated for specificity showing that there is no interference with the ibuprofen peak. Recovery was 85-90% for ibuprofen. The calibration curve was linear over the concentration 0.5-10 ug/ml.

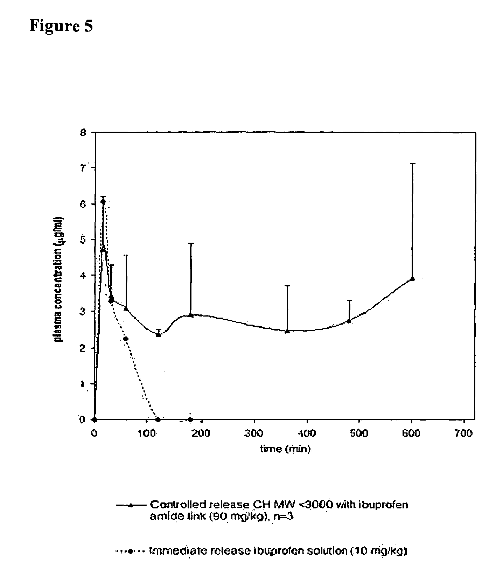

Figure 5 shows a controlled release behavior for the formula of ibuprofen conjugated with chitosan and an immediate release behavior for formula unconjugated with chitosan.

Above results illustrate that ibuprofen shows a zero order drug release as it passes the gastrointestinal tract when given as a covalent conjugate with chitosan.

### Example 4

### Taste masking

### Taste mask evaluation test of ibuprofen covalently conjugated by oligosaccharide chitosan

Five solutions were evaluated for their taste. Six randomly chosen human subjects evaluated the taste. Each subject tasted about 0.5 ml of each solution. A sufficient wash out period was considered between each individual solution.

### Solution 1 (S1): contains placebo (0.01 M HCl and chitosan 1 % w/w)

Solution 2 (S2): Ibuprofen (final concentration around 200 mg/ 5 ml) and chitosan (1% w/w) physical mixture in 0.01 M HCl solution.

Solution 3 (S3): Ibuprofen (final concentration around 200 mg/5 ml) chitosan (1% w/w) chemical conjugate in 0.01 M HCl solution.

3 types of tastes could be differentiated. First: the slight acidity of the solvent, second: the mild astringent taste of the chitosan and third: the high irritation taste of ibuprofen.

The tastes were then graded from 0 where there is no taste to 5 where unacceptable taste appears, according to Table 1

**Table 1: Grading system used for taste evaluation**

| **Taste** | **Degree** |
|---|---|
| Acidity | 0 to 5 |
| Astringent | 0 to 5 |
| Irritation | 0 to 5 |

**Table 2: Summary of results of taste sensation for the four solutions**

| | Acidity | | | Astringent | | | Irritation | | |
|---|---|---|---|---|---|---|---|---|---|
| Solution Subject | S1 | S2 | S3 | S1 | S2 | S3 | S1 | S2 | S3 |
| M-1 | 0 | 1 | 0 | 3 | 2 | 0 | 0 | 4 | 0 |
| I-2 | 0 | 2 | 1 | 0 | 0 | 1 | 0 | 4 | 0 |
| H-3 | 0 | 0 | 2 | 0 | 0 | 0 | 1 | 5 | 0 |
| N-4 | 0 | 3 | 1 | 3 | 3 | 2 | 0 | 5 | 0 |
| F-5 | 2 | 3 | 3 | 2 | 2 | 3 | 1 | 5 | 3 |
| G-6 | 0 | 0 | 1 | 3 | 0 | 1 | 0 | 3 | 0 |
| % average | 6.67 | 30.00 | 26.67 | 36.67 | 26.67 | 23.33 | 6.67 | 86.67 | 10.00 |
| % total average | **S1** | **S2** | **S3** | | | | | | |
| | **16.67** | **47.78** | **20.00** | | | | | | |

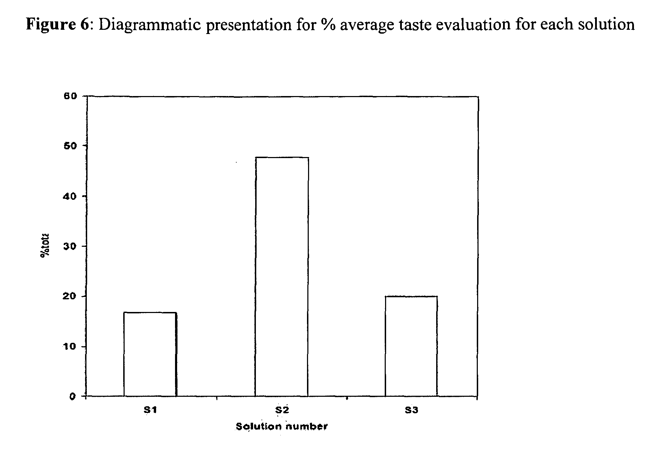

Solution S3 containing the conjugate of ibuprofen and chitosan was found to have average sensation almost close to S 1 (placebo) and was far from the high irritant sensation of physicial mixture, as shown in table 2 and Fig. 6. This indicates a masking effect upon conjugating ibuprofen with the chitosan oligosaccharide polymer.

The features disclosed in the foregoing description, in the claims and in the drawings may, both separately and in any combination thereof, be material for realizing the invention in divers forms thereof.

## Claims

1. Composition comprising an aqueous solvent and a conjugate of at least one hydrophilic polymer having a primary amine functional group, a derivative or monomer thereof and at least one acidic drug.

2. Composition according to claim 1, wherein the polymer is chitosan or the monomer is glucosamine.

3. Composition according to claim 1 or 2, wherein the acidic drug contains an acidic functional group selected from the group consisting of carboxylic, sulfonic, nitric, phosphoric group, or a salt thereof.

4. Composition according to any of the preceding claims, wherein the acidic drug is insoluble in water.

5. Composition according to claim 1, wherein the acidic drug is selected from beta lactam antibiotics, quinolone antibiotics, sulfonamide, nonsteroidal anti-inflammatory drugs, antihyperlipidimic drugs, psychostimulants, prostaglandin vasodilators, antidepressants, anticonvulsive agents, hemostatic agents, antituberculosis agents, hepatic protectant agents, flavoring agents, sweeteners, antispasmodic agents, antineoplastic agents, antiseptic agents, antiinflammatory cortisone drugs, hypoglycemic drugs, antiarrythmetic agents, antihypertensive agents, antiallergy agents, emollients, agents for gastrointestinal disorders and anti-infective.

6. Composition according to claim 5, wherein the acidic drug is selected from cephalosporins, penicillins, nalidixic acids, ciprofloxacin, acediasulfone, amfenac, diclofenac, ibuprofen, indomethacin, acetyl salicylic acid, clinadac, naproxen, mefenamic acid, fosfomycin, bendazac, acifran, fluvastatin, atorvastatin, aceglutamide, alprostadil, amineptine, gamma aminobutyric acid, gabapentin, valproic acid, aminocaproic acid, aminosalicylic acid, amino acid arginine, aspartic acid, betaine, aspartam, baclofen, bendamustine, bromebric acid, sodium borocaptate, chlorambucil, methotrexate, bismuth subgallate, dodicin, betamethasone, calcium mesoxalate, capobenic acid, captopril, cromolyn sodium, spaglumic acid, docusate sodium, mesalamine and flumequine.

7. Composition according to any of the preceding claims, wherein the aqueous solvent has a pH of 4 to 7, preferably 4 to 6.5.

8. Composition according to claim 7, wherein the aqueous solvent is 0.01 M HCl solution.

9. Composition according to any of the preceding claims, further comprising at least one additional therapeutically active ingredient other than the acidic drug as disclosed in claim 1.

10. Composition according to any of the preceding claims, additionally comprising pharmaceutical excipients selected from the group consisting of sweeteners, coloring agents, preservatives, thickeners and flavoring agents.

11. Method for preparing a conjugate of at least one hydrophilic polymer having a primary amine functional group, a derivative or monomer thereof and at least one acidic drug, comprising the steps of reacting the acidic drug to obtain an acid halide, ester, anhydride or other intermediate product and reacting the intermediate product with the hydrophilic polymer to form a conjugate via amide bond formation.

12. Method according to claim 11, wherein the method is carried out under acidic environment.

13. Use of the composition according to any of the claims 1 to 10, for oral administration.

14. Use of the composition according to any of the claims 1 to 10 for taste masking of the acidic drug.
